(19) Europäisches Patentamt / European Patent Office / Office européen des brevets

(11) **EP 2 676 624 B1**

(12) **EUROPÄISCHE PATENTSCHRIFT**

(45) Veröffentlichungstag und Bekanntmachung des Hinweises auf die Patenterteilung:
**14.12.2016 Patentblatt 2016/50**

(21) Anmeldenummer: **12172428.0**

(22) Anmeldetag: **18.06.2012**

(51) Int Cl.:
*A61B 18/12* *(2006.01)*

(54) **Hochfrequenz-Chirurgiegerät**

High frequency surgical device

Appareil chirurgical haute fréquence

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**25.12.2013 Patentblatt 2013/52**

(73) Patentinhaber: **ERBE Elektromedizin GmbH 72072 Tübingen (DE)**

(72) Erfinder: **Hagg, Martin 72827 Wannweil (DE)**

(74) Vertreter: **Bohnenberger, Johannes et al Meissner Bolte Patentanwälte Rechtsanwälte Partnerschaft mbB Postfach 86 06 24 81633 München (DE)**

(56) Entgegenhaltungen:
EP-A2- 0 194 078      WO-A1-96/14021
US-A- 3 478 744       US-A1- 2008 039 832
US-A1- 2011 071 518

• **Jeffrey Eggleston ET AL: "Electrosurgical Devices" In: "The Biomedical Engineering Handbook, Second Edition. 2 Volume Set", 28. Dezember 1999 (1999-12-28), CRC Press, XP055053360, ISSN: 1097-9409 ISBN: 978-1-42-004951-0 Bd. 19991228DOI: 10.1201/9781420049510.ch81, * Tabelle 81.3 ***

EP 2 676 624 B1

**Beschreibung**

Die Erfindung betrifft ein Hochfrequenz-Chirurgiegerät (HF-Chirurgiegerät) gemäß dem Oberbegriff des Anspruchs 1.

Ein HF-Chirurgiegerät der hier angesprochenen Art ist beispielsweise aus der DE 10 2008 004 241 A1 bekannt. Ein weiteres HF-Chirurgiegerät ist aus der EP 0 194 078 A2 bekannt. Die Fig. 6 der vorliegenden Anmeldung zeigt eine Prinzipskizze eines bekannten HF-Chirurgiegeräts 1 gemäß DE 10 2008 004 241 A1. Es umfasst einen HF-Generator 3, der ein hochfrequentes Ausgangssignal a erzeugt, insbesondere einen hochfrequenten Wechselstrom, der einem mit dem HF-Generator 3 elektrisch verbundenen HF-chirurgischen Instrument zugeführt wird. Bei dem HF-chirurgischen Instrument kann es sich beispielsweise um ein Instrument zur Argon-Plasma-Koagulation oder zum HF-Schneiden handeln, welches mittels einer oder mehr aktiven Elektroden auf das zu behandelnde biologische Gewebe 5 eines Patienten einwirkt. Das zu behandelnde Gewebe 5 des Patienten wirkt dabei als Lastimpedanz, die je nach Behandlungsgrad veränderlich ist. Das HF-Chirurgiegerät 1 umfasst darüber hinaus ein Leistungsnetzteil 7, welches die Netzspannung (50 Hz AC) in eine für den HF-Generator geeignete Spannung umwandelt.

Üblicherweise wird in dem HF-Generator 3 zunächst die Hochfrequenzleistung mit einer Träger- bzw. Grundfrequenz erzeugt und in nachgeschalteten Verstärkerstufen verstärkt. Je nach Anwendungsfall wird das von dem HF-Generator 3 erzeugte, die Grundfrequenz aufweisende Ausgangssignal im zeitlichen Verlauf insbesondere mit einem variablem Tastverhältnis moduliert. Hierzu kann entweder mittels einer Modulationseinrichtung 9 unmittelbar auf den HF-Generator 3 derart eingewirkt werden, dass durch ein Pulsen bzw. Tasten der Hochfrequenzleistung, d.h. durch entsprechendes An- und Ausschalten des Ausgangssignals dasselbe moduliert wird. Es handelt sich somit um eine Pulsweitenmodulation (PWM), wobei die Pulsfrequenz bzw. die Taktfrequenz den Modulationsgrad des Ausgangssignals bestimmt. Die Modulationsfrequenz $f_{\text{Modulation}}$ ergibt sich dabei durch die folgende Formel:

$$f_{\text{Modulation}} = 1/T,$$

wobei T die Periodendauer des rechteckförmigen Modulationssignals ist. Hierzu kann der HF-Generator beispielsweise eine Schaltungsanordnung mit MOSFET-Transistoren aufweisen, die abwechselnd ein- und ausgeschaltet werden. Die Ansteuerung der Modulationseinrichtung 9 erfolgt dabei üblicherweise durch eine Steuereinrichtung 11.

Eine weitere bekannte Möglichkeit, das Ausgangssignal zu modulieren, ist eine direkte Ansteuerung des Leistungsnetzteils 7 mittels der Steuereinrichtung 11. Hierdurch wird es möglich, die Amplitude des Ausgangssignals des HF-Generators 3 zu modulieren, wobei die Steuereinrichtung 11 eine Wirkung als Modulationseinrichtung 9 aufweist.

Die Grundfrequenz des HF-Generators liegt üblicherweise bei ca. 300 bis 500 kHz. Die Modulation erfolgt üblicherweise mit Frequenzen zwischen 1 kHz und 50 kHz. Vorzugweise kommen jedoch Modulationsfrequenzen von größer als 20 kHz zum Einsatz, um die Erzeugung von unangenehmen Geräuschen zu vermeiden. Beispielsweise bei der "gepulsten Argon-Plasma-Koagulation" können aber auch niedrigere Modulationsfrequenzen im Hz-Bereich zum Einsatz kommen.

Generell besteht bei HF-Chirurgiegeräten die Vorgabe, dass niederfrequente Stromanteile, die über den Patienten fließen können und folglich neuromuskuläre Stimulationen (z.B. Muskelzucken) auslösen können, auf sehr geringe Werte beschränkt sein müssen. Sofern diese Vorgabe erfüllt ist, dürfen bestimmte Anwendungsteile als CF (Cardiac Floating) bezeichnet werden. Ein Nachteil dieser herkömmlichen geringen Modulationsfrequenzen liegt darin, dass sie unterhalb der Grenze liegen, ab der nach heutigen wissenschaftlichen Erkenntnissen keine neuromuskulären Phänomene, wie z.B. Muskelzucken, mehr auftreten. Es wurde festgestellt, dass diese Grenze bei ca. 100 kHz liegt. Eine einschlägige Norm IEC 60601-2-2 regelt deshalb, dass die Grundfrequenz der HF-Chirurgiegeräte größer als 200 kHz sein muss. Hierbei wird jedoch übersehen, dass die niedrigen Modulationsfrequenzen auch bei einer Grundfrequenz von größer als 200 kHz berücksichtig werden müssen, weil diese im Frequenzspektrum des gesamten Ausgangssignals deutlich hervortreten und damit neuromuskuläre Phänomene hervorrufen können.

Aus dem Stand der Technik ist es zwar bekannt, zur Vermeidung von neuromuskulären Reizungen durch niederfrequente und damit neuromuskulär wirksame Modulationssignale, so genannte Auskoppelkondensatoren im Patientenkreis vorzusehen, diese können aber eine insbesondere durch das Funkenspiel zwischen der aktiven Elektrode des chirurgischen Instruments und dem Gewebe bedingte neuromuskuläre Reizung nicht vollständig verhindern.

Aufgabe der vorliegenden Erfindung ist es daher, ein HF-Chirurgiegerät zu schaffen, welches das Auftreten von neuromuskulären Phänomenen sicher vermeidet.

Zur Lösung dieser Aufgabe wird ein HF-Chirurgiegerät mit den Merkmalen des Anspruchs 1 vorgeschlagen.

Das Hochfrequenz-Chirurgiegerät umfasst einen Hochfrequenz-Generator, der ein hochfrequentes Ausgangssignal zur Behandlung, insbesondere zum Schneiden oder Koagulieren von biologischem Gewebe erzeugt, wobei der Hochfrequenz-Generator so ausgebildet ist, dass sein Ausgangssignal eine vorbestimmte Grundfrequenz aufweist, und wobei eine Modulationseinrichtung vorgesehen ist, die zum Modulieren des Ausgangssignals mit einer Modulationsfrequenz dient, wobei die Modulationsfrequenz kleiner ist als die Grundfrequenz. Das Hochfrequenz-Chirurgiegerät zeichnet sich dadurch aus, dass die Modulationsfrequenz wenigstens 100 kHz beträgt, und dass das Ausgangssignal durch eine Pulsweitenmodulation derart modulierbar ist, dass sich ein für eine spezifische Anwendung des Hochfrequenz-Chirurgiegeräts geeigneter Crest-Faktor des modulierten Ausgangssignals einstellt, wobei ein Crest-Faktor im Bereich von 5 bis 11 oder größer als 15 einstellbar ist.

Ein wesentlicher Punkt der Erfindung liegt also darin, dass neuromuskuläre Phänomene dadurch ausgeschlossen werden können, dass die Modulationsfrequenz 100 kHz nicht unterschreitet. Zu beachten gilt es jedoch, dass der Frequenzbandabstand zwischen der Modulationsfrequenz und der Grundfrequenz des HF-Generators nicht zu klein sein darf, weil sonst der Crest-Faktor nicht groß genug für einen bestimmten Anwendungsfall des HF-Chirurgiegeräts eingestellt werden kann, wobei der Crest-Faktor das Verhältnis von der Spitzenamplitude des Ausgangssignals zu dessen Effektivwert beschreibt. Wenn also der Modulationsgrad des Ausgangssignals und die Grundfrequenz nicht in geeigneter Weise aufeinander abgestimmt sind, kann das Ziel einer größeren Spitzenspannung bei kleiner Effektivspannung, d.h. ein großer Crest-Faktor nicht mehr erreicht werden. Dem Crest-Faktor CF kommt in der HF-Chirurgie eine besondere Bedeutung zu, da er mathematisch den Modulationsgrad des Ausgangssignals des HF-Generators beschreiben kann. Je nach Einsatzgebiet des HF-Generators sind unterschiedliche Modulationsgrade und damit Crest-Faktoren des Ausgangssignals zu realisieren. Um beispielsweise für eine Gewebe-Koagulation einen geeigneten Koagulationsgrad von beispielsweise CF = 2,5 zu erhalten, muss der Modulationsgrad des Ausgangsstroms des HF-Generators folglich entsprechend eingestellt werden. Gemäß der Erfindung werden daher die Grundfrequenz des HF-Generators und der Modulationsgrad des Ausgangssignals derart aufeinander abgestimmt, dass ein geeigneter, d.h. für eine bestimmte Anwendung ausreichend großer Crest-Faktor unter der Bedingung resultiert, dass die Modulationsfrequenz 100 kHz nicht unterschreitet.

Das Ausgangssignal ist mittels einer Pulsbreitenmodulation moduliert. Bei einer Ausführungsform der Erfindung ist vorgesehen, dass die Modulationsfrequenz und die Grundfrequenz konstant sind. Bei einer anderen Ausführungsform der Erfindung kann hingegen vorgesehen sein, dass die Modulationsfrequenz variabel ist, während die Grundfrequenz zur Veränderung des Crest-Faktors konstant ist. Weiterhin kann vorgesehen sein, dass ein geeigneter Crest-Faktor durch Variation des Tastverhältnisses des Modulationssignals einstellbar ist. Dies ist insbesondere dann von Vorteil, wenn die Modulationsfrequenz konstant gehalten wird.

Der Crest-Faktor wird vorzugsweise anhand des Ausgangssignals bestimmt und soll einen vorbestimmten Mindestwert vorzugsweise nicht unterschreiten. Es kann eine entsprechende Erfassungseinrichtung vorgesehen sein, welche die Modulationsfrequenz, die Grundfrequenz des Generators und den Crest-Faktor erfasst.

Bei einer vorgegebenen gewünschten Modulationsfrequenz, die größer als 100 kHz ist, wird folglich die Modulation der Grundfrequenz so an die Modulationsfrequenz angepasst, dass ein vorbestimmter einzustellender Crest-Faktor erzielt wird. Beispielsweise kann ein Crest-Faktor für eine effektive Kontakt-Koagulation und/oder für eine kontaktlose Koagulation mit geringer Schneidwirkung eingestellt werden. Es versteht sich also, dass der Crest-Faktor in Abhängigkeit von dem jeweiligen Einsatz des Chirurgiegeräts, beispielsweise zum Schneiden und/oder Koagulieren von Gewebe oder dergleichen HF-chirurgische Behandlung, variieren kann.

Besonders bevorzugt wird ein Hochfrequenzgenerator, bei dem die Modulationsfrequenz gleich oder größer als 200 kHz ist. Entsprechend muss der Wert der Grundfrequenz größer sein, insbesondere deutlich größer als 500 kHz. Besonders vorteilhaft ist es, wenn die Grundfrequenz größer ist als das Fünffache der Modulationsfrequenz. Insbesondere können gemäß der Erfindung die Grundfrequenzen im ein- oder gar mehrstelligen Megahertz-Bereich liegen.

Weitere vorteilhafte Merkmale des erfindungsgemäßen Verfahrens ergeben sich aus den Unteransprüchen.

Die Erfindung wird im Folgenden anhand der Zeichnung näher erläutert. Es zeigen:

Fig. 1 Eine schematische Darstellung eines Ausgangssignals eines HF-Generators in Form einer kontinuierlichen Sinusspannung;

Fig. 2 eine schematische Darstellung eines modulierten Ausgangssignals mit einem gegenüber dem unmodulierten Ausgangssignal veränderten Crest-Faktor;

Fig. 3 eine schematische Darstellung eines modulierten Ausgangssignals des HF-Generators mit einem gegenüber dem unmodulierten Ausgangssignal noch weiter veränderten Crest-Faktor;

Fig. 4 eine schematische Darstellung eines modulierten Ausgangssignals des HF-Generators mit einem gegenüber dem unmodulierten Ausgangssignal noch weiter veränderten Crest-Faktor;

Fig. 5 eine schematische Darstellung eines modulierten Ausgangssignals des HF-Generators mit einem gegenüber dem unmodulierten Ausgangssignal veränderten Crest-Faktor, und

Fig. 6    eine schematische Darstellung eines aus dem Stand der Technik, beispielsweise der DE 10 2008 004 241 A1 bekannten HF-Chirurgiegeräts. Die Bezugzeichen der Figur 6 werden auch verwendet um ein erfindungsgemäßes Hochfrequenzchirurgiegerät zu erläutern.

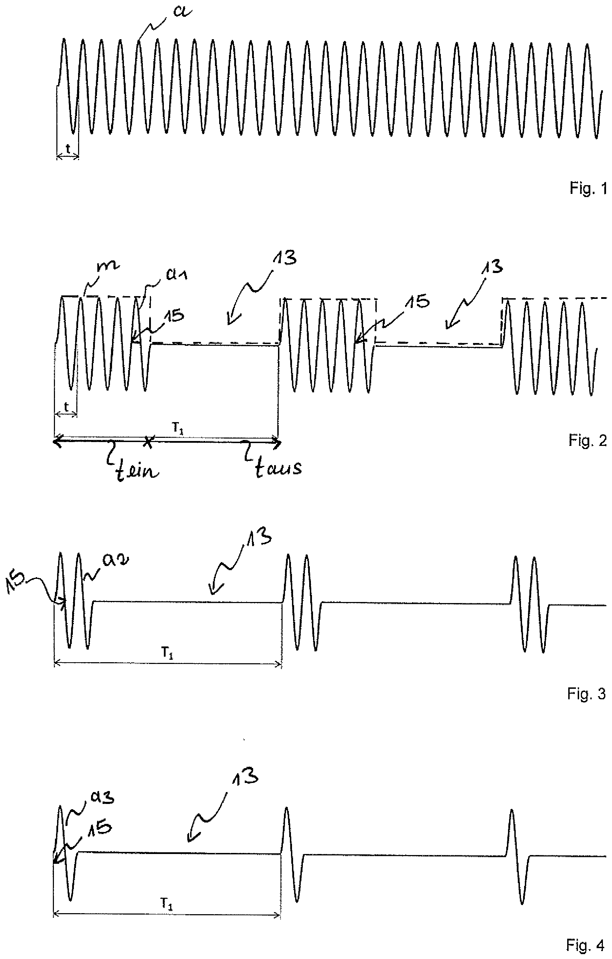

Die Fig. 1 zeigt ein Ausgangssignal a mit einer Generatorfrequenz $f_{Generator}$, die sich aus der folgenden Formel ergibt:

$$f_{Generator} = 1/t,$$

wobei t die Periodendauer des Ausgangssignals a ist, und wobei das Ausgangssignal a im vorliegenden Fall als Ausgangsspannung dargestellt ist. Das in Fig. 1 gezeigte Ausgangssignal a eines HF-Generators 3 ist in seiner ursprünglichen Form, d.h. nicht moduliert dargestellt. Das kontinuierliche Sinussignal gemäß Fig. 1 weist beispielhaft einen Crest-Faktor von CF=1,41 auf.

Die Fig. 2 bis 4 zeigen schematische Darstellungen von modulierten Ausgangssignalen a1, a2 und a3, bei denen die Grundfrequenz eines HF-Generators 3 $f_{Generator}$ konstant ist und mit der in Fig. 1 gezeigten Grundfrequenz übereinstimmt. Im Gegensatz zu dem in Fig. 1 gezeigten Ausgangssignal a sind die Ausgangssignale a1, a2 und a3 der Fig. 2 bis 4 jedoch durch eine entsprechende Modulationseinrichtung 9 und ein entsprechendes Modulationssignal m moduliert. Die gewählte Modulationsform ist hier eine Pulsweitenmodulation, die mittels des rechteckförmigen Modulationssignals m bewerkstelligt wird. Das rechteckförmige Modulationssignal m weißt eine Einschaltzeit $t_{ein}$ und eine Ausschaltzeit $t_{aus}$ auf, die zusammen die Periodendauer $T_1$ des Modulationssignals ergeben. Durch die Ein- und Ausschaltzeiten des Ausgangssignals a1 ergeben sich Pulspausen 13 und Pulspakete 15, die durch die Pulspausen voneinander getrennt sind. Aus der Periodendauer $T_1$ des Modulationssignals m ergibt sich wiederum die Modulationsfrequenz, die sich nach der folgenden Formel berechnen lässt:

$$f_{Modulation} = 1/T_1.$$

Aus dem Verhältnis der Einschaltzeit zu der Summe aus Einschalt- und Ausschaltzeit des Modulationssignals m lässt sich das sogenannte Tastverhältnis (Duty Cyle) D bestimmen:

$$D = t_{ein}/(t_{ein}+t_{aus}) = t_{ein}/T_1.$$

Die Fig. 2 zeigt ein pulsbreitenmoduliertes Ausgangssignal a1. Mit anderen Worten wurde das Ausgangssignal a1 durch einen Rechteckimpuls moduliert, der eine Periodendauer $T_1$ aufweist, die derart gemäß der Erfindung gewählt wurde, dass die resultierende Modulationsfrequenz $f_{Modulation}=1/T_1 \geq 100$ kHz beträgt. Es wird deutlich, dass der Crest-Faktor CF des in Fig. 2 gezeigten Ausgangssignals a1 des HF-Generators 3 größer ist als der Crest-Faktor des in Fig. 1 gezeigten unmodulierten Ausgangssignals a, da die Pulspausen 13 zwischen den Pulspaketen 15 für eine Reduzierung des Effektivwerts des Ausgangssignals a1 sorgen. Der Crest-Faktor, der sich aus dem Verhältnis des Spitzenwertes, d.h. der maximalen Amplitude zum Effektivwert des Ausgangssignals ergibt, wird folglich bei einer Reduzierung des Effektivwerts des Ausgangssignals a1 größer.

Die Fig. 3 zeigt eine schematische Darstellung eines weiteren modulierten Ausgangssignals a2, welches eine abgeänderte Pulsweitenmodulation derart aufweist, dass ein im Vergleich zu den Fig. 1 und 2 vergrößerter Crest-Faktor CF resultiert. Wie bei der Fig. 2 ist auch bei dem Ausgangssignal a2 gemäß Fig. 3 die Grundfrequenz des Generators $f_{Generator}$ unverändert und auch die Modulationsfrequenz $f_{Modulation}$ ist gemäß der Erfindung größer oder gleich 100 kHz gewählt und entspricht der in Fig. 2 gezeigten Modulationsfrequenz, was sich in den übereinstimmenden Periodendauern $T_1$ zeigt.

Die Vergrößerung des Crest-Faktors CF wird bei dem Ausgangssignal a2 gemäß Fig. 3 dadurch erreicht, dass das Tastverhältnis D verändert wird, während die Modulationsfrequenz $f_{Modulation}$ konstant $\geq 100$ kHz bleibt. Die Fig. 3 macht dabei deutlich, dass die Pulspausen 13 zwischen den Pulspaketen 15 größer sind als bei dem Beispiel gemäß Fig. 2. Dies wird dadurch erreicht, dass die Einschaltzeit $t_{ein}$ verkleinert wird, während die Ausschaltzeit $t_{aus}$ des Rechteckimpulses m vergrößert wird. Durch diese Vergrößerung der Pulspause 13 zwischen den Pulspaketen 15 ergibt sich eine Verkleinerung des Effektivwerts des Ausgangssignals a2, so dass der Crest-Faktor CF erhöht wird. Mit anderen Worten wird durch die Vergrößerung der Pulspause 13 die Anzahl der Perioden t je Pulspaket 15 verringert.

Die Fig. 4 zeigt noch ein weiteres Beispiel für ein moduliertes Ausgangssignal a3 des HF-Generators 3, welches einen gegenüber den Fig. 1 bis 3 noch vergrößerten Crest-Faktor CF aufweist. Wie auch bei den Fig. 2 und 3 wurde die Grundfrequenz $f_{Generator}$ des HF-Generators 3 nicht verändert, was sich an der gleichbleibenden Periodendauer t des Ausgangssignals in den Pulspaketen 15 zeigt. Entsprechend wurde auch die Modulationsfrequenz $f_{Modulation}$ gegenüber den in den Fig. 2 und 3 gezeigten Modulationen nicht verändert, was wiederum die gleich bleibende Periodendauer $T_1$ zeigt. Nichtsdestotrotz weist das in Fig. 4 gezeigte modulierte Ausgangssignal a3 einen gegenüber den Fig. 2 und 3 noch vergrößerten Crest-Faktor CF auf.

Die weitere Vergrößerung des Crest-Faktors wird dadurch erreicht, dass die Pulspausen 13 zwischen den Pulspaketen 15 noch weiter vergrößert werden, während die Periodendauern t je Pulspaket 15 weiter verringert werden. In der Fig. 4 weist ein Pulspaket 5 nur noch eine Periode t des Ausgangssignals a3 auf. Hierdurch wird wiederum der Effektivwert des Ausgangssignals a3 verkleinert, so dass der Crest-Faktor CF gegenüber den in den Fig. 2 und 3 gezeigten Beispielen noch größer wird.

Auch in den Fig. 3 und 4 erfolgt somit eine Veränderung des Tastverhältnisses D mittels einer Pulsbreitenmodulation derart, dass bei einer feststehenden Grundfrequenz $f_{Generator}$ des HF-Generators 3 und bei einer feststehenden Modulationsfrequenz $f_{Modulation}$ der Crest-Faktor an verschiedenste Anwendungen, wie beispielsweise eine HF-Koagulation oder ein HF-Schneidvorgang anpassbar ist.

Insgesamt lässt sich somit festhalten, dass bei gleichbleibender Grundfrequenz $f_{Generator}$ und gleichbleibender Modulationsfrequenz $f_{Modulation}$ der Crest-Faktor durch eine Reduzierung des Tastverhältnisses D des Modulationssignals vergrößert werden kann. Hierzu ist es notwendig, dass die Grundfrequenz und die Modulationsfrequenz derart gewählt werden, dass Crest-Faktoren in einem ausreichend großen Bereich durch eine Pulsweitenmodulation einstellbar sind. Dies erfordert einen relativ großen Abstand zwischen der Grundfrequenz $f_{Generator}$ und der Modulationsfrequenz $f_{Modulation}$, wobei die Randbedingung $f_{Modulation} \geq 100$ kHz erfüllt sein muss, um neuromuskuläre Stimulationen sicher zu vermeiden. Die Grundfrequenz beträgt zur Erfüllung der oben genannten Anforderungen vorzugsweise das Fünffache der Modulationsfrequenz.

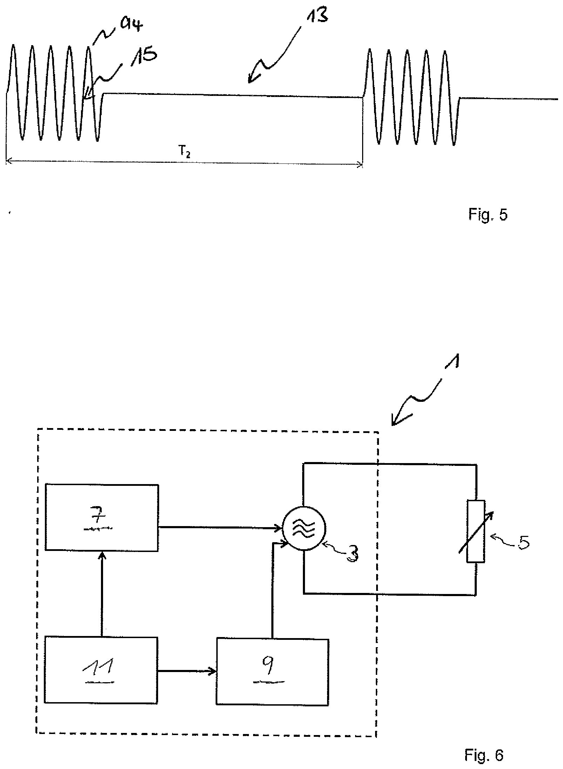

In der Fig. 5 ist eine weitere Ausführungsform der Erfindung gezeigt, bei welcher die Grundfrequenz $f_{Generator}$ des Ausgangssignals a4 unverändert ist, während eine Erhöhung des Crest-Faktors CF durch eine Reduzierung der Modulationsfrequenz $f_{Modulation}$ erzeugt wird, anders als in der in Fig. 2-4 gezeigten Variante. Demnach ist die Periodendauer $T_2$ des Modulationssignals gemäß Fig. 5 größer als die in Fig. 2 verwendete Periodendauer $T_1$, wobei die Perioden t der Pulspakete 15 bei den Ausführungsformen der Fig. 2 und 5 übereinstimmen. Anstatt jedoch die Modulationsfrequenz $f_{Modulation}$, wie in den Fig. 2 bis 4 gezeigt, konstant zu halten und lediglich die Perioden t je Pulspaket 15 zu reduzieren bzw. die Pulspausen 13 zu erhöhen, wurde in der Fig. 5 lediglich die Pulspause 13 erhöht, ohne die Periodenzahl je Pulspaket 15 zu verringern oder zu erhöhen. Es zeigt sich somit, dass eine Erhöhung des Crest-Faktors auch dadurch erfolgen kann, dass die Modulationsfrequenz $f_{Modulation}$ verringert wird.

Auch bei dieser Ausführungsform der Erfindung ist es jedoch notwendig, dass die Randbedingung $f_{Modulation} \geq 100$ kHz erfüllt ist, um neuromuskuläre Stimulationen sicher zu vermeiden. Daher muss auch bei dieser Ausführungsform ein ausreichend großer Abstand zwischen der Grundfrequenz des Generators und der Modulationsfrequenz gegeben sein, um Crest-Faktoren in einem ausreichend großen Bereich durch eine Variation der Modulationsfrequenz im Bereich $\geq 100$ kHz realisieren zu können.

Insgesamt zeigen die Fig. 2 bis 5, dass die Grundfrequenz des Generators gegenüber der Modulationsfrequenz um ein Mehrfaches erhöht sein muss, um entsprechend große und variable Crest-Faktoren, insbesondere im Bereich von 5 bis wenigstens 11, für verschiedenste Anwendungsfälle erzeugen zu können. Weiterhin gibt es Anwendungsfälle, z.B. bei der kontaktlosen Spray-Koagulation, bei denen Crest-Faktoren von 15 oder sogar noch größer benötigt werden, um den gewünschten Behandlungseffekt zu erzielen.

Entscheidend ist für die vorliegende Erfindung, dass nicht nur die Grundfrequenz des Generators größer als 100 kHz ist, nämlich insbesondere in einem Bereich zwischen 300 bis 600 kHz liegt, sondern dass die Modulationsfrequenz $f_{Modulation}$ 100 kHz nicht unterschreitet. Der Crest-Faktor, der für einen bestimmten Anwendungsfall des HF-Chirurgiegeräts 1 eingestellt werden soll, muss dann über ein geeignetes Modulationsverfahren unter der Bedingung eingestellt werden, dass die Modulationsfrequenz 100 kHz nicht unterschreitet. Wie gesagt ist es hierfür notwendig, dass die Grundfrequenz des Generators 3 die Modulationsfrequenz um ein Mehrfaches übersteigt. Insbesondere können gemäß der Erfindung die Grundfrequenzen im ein- oder gar mehrstelligen Megahertz-Bereich liegen.

Insgesamt zeigt sich somit, dass durch die vorliegende Erfindung das Auftreten von neuromuskulären Stimulationen sicher vermieden wird, indem die Modulationsfrequenz mindestens 100 kHz beträgt und folglich oberhalb der Grenze liegt, ab der nach heutigen wissenschaftlichen Erkenntnissen keine neuromuskulären Phänomene, wie z.B. Muskelzucken, mehr auftreten. Gleichzeitig wird ein geeigneter Crest-Faktor für einen spezifischen Anwendungsfall, wie der Kontakt-Koagulation oder eines Schneidvorgangs, dadurch erzielt, dass die Grundfrequenz wesentlich größer ist als die üblichen 350 kHz. Dadurch wird ein ausreichender Abstand zwischen den Frequenzbändern der Modulationsfrequenz und der Grundfrequenz geschaffen, der die Erzeugung eines ausreichend großen Crest-Faktors erlaubt, der insbesondere größer als 15 ist.

Bezugszeichenliste

| 1 | HF-Chirurgiegerät |
| 3 | HF-Generator |
| 5 | Biologisches Gewebe |
| 7 | Leistungsnetzteil |
| 9 | Modulationseinrichtung |
| 11 | Steuereinrichtung |

13          Pulspausen
15          Pulspakete
a, a1 bis a3   Ausgangssignal
$t_{ein}$         Einschaltzeit
$t_{aus}$         Ausschaltzeit
$T_1, T_2$      Modulationsperiode
t           Ausgangssignalperiode

**Patentansprüche**

1.  Hochfrequenz-Chirurgiegerät (1) mit einem Hochfrequenz-Generator (3), der ein hochfrequentes Ausgangssignal (a) zur Behandlung von biologischem Gewebe (5) erzeugt und so ausgebildet ist, dass sein Ausgangssignal (a) eine vorbestimmte Grundfrequenz ($f_{Generator}$) aufweist, wobei eine Modulationseinrichtung (9, 11) vorgesehen ist, die zum Modulieren des Ausgangssignals (a) mit einer Modulationsfrequenz ($f_{Modulation}$) dient, wobei die Modulationsfrequenz ($f_{Modulation}$) kleiner ist als die Grundfrequenz und wenigstens 100 kHz beträgt, und wobei das Ausgangssignal (a) durch eine Pulsweitenmodulation derart modulierbar ist, dass sich ein für eine spezifische Anwendung des Hochfrequenz-Chirurgiegeräts (1) geeigneter Crest-Faktor (CF) des modulierten Ausgangssignals (a1,..., a4) einstellt,
    **dadurch gekennzeichnet, dass**
    ein Crest-Faktor (CF) im Bereich von 5 bis 11 oder größer als 15 einstellbar ist.

2.  Hochfrequenz-Chirurgiegerät nach Anspruch 1,
    **dadurch gekennzeichnet, dass**
    die Modulationsfrequenz (modulation) und die Grundfrequenz ($f_{Generator}$) konstant sind.

3.  Hochfrequenz-Chirurgiegerät nach Anspruch 1,
    **dadurch gekennzeichnet, dass**
    die Modulationsfrequenz ($f_{Modulation}$) variabel und die Grundfrequenz ($f_{Generator}$) konstant ist zur Veränderung des Crest-Faktors.

4.  Hochfrequenz-Chirurgiegerät nach einem der vorhergehenden Ansprüche,
    **dadurch gekennzeichnet, dass**
    die Modulationsfrequenz größer als 200 kHz ist.

5.  Hochfrequenz-Chirurgiegerät nach einem der vorhergehenden Ansprüche,
    **dadurch gekennzeichnet, dass**
    die Grundfrequenz ($f_{Generator}$) größer als das Fünffache der Modulationsfrequenz ($f_{Modulation}$) ist.

**Claims**

1.  Electrosurgical device (1) comprising a radiofrequency generator (3) which generates a radiofrequency output signal (a) for treating biological tissue (5) and is embodied in such a way that the output signal (a) thereof has a predetermined fundamental frequency ($f_{Generator}$), wherein provision is made of a modulation apparatus (9, 11) which serves to modulate the output signal (a) with a modulation frequency ($f_{Modulation}$), wherein the modulation frequency ($f_{Modulation}$) is smaller than the fundamental frequency and is at least 100 kHz, and wherein the output signal (a) is able to be modulated by a pulse-width modulation in such a way that a crest factor (CF) of the modulated output signal (al,..., a4) sets in, said crest factor being suitable for a specific application of the electrosurgical device (1),
    **characterized in that**
    a crest factor (CF) is adjustable in the range of 5 to 11 or greater than 15.

2.  Electrosurgical device according to Claim 1,
    **characterized in that**
    the modulation frequency ($f_{Modulation}$) and the fundamental frequency ($f_{Generator}$) are constant.

3.  Electrosurgical device according to Claim 1,
    **characterized in that**
    the modulation frequency ($f_{Modulation}$) is variable and the fundamental frequency ($f_{Generator}$) is constant for the purposes of modifying the crest factor.

4.  Electrosurgical device according to one of the preceding claims,
    **characterized in that**
    the modulation frequency is greater than 200 kHz.

5.  Electrosurgical device according to one of the preceding claims,
    **characterized in that**
    the fundamental frequency ($f_{Generator}$) is greater than five times the modulation frequency ($f_{Modulation}$).

**Revendications**

1.  Appareil chirurgical à haute fréquence (1) comportant un générateur à haute fréquence (3) qui génère un signal de sortie à haute fréquence (a) pour traiter des tissus biologiques (5) et est conçu de manière à ce que son signal de sortie (a) présente une fréquence fondamentale ($f_{générateur}$) prédéterminée, dans lequel il est prévu un dispositif de modulation (9, 11) qui est utilisé pour moduler le signal de sortie (a) avec une fréquence de modulation ($f_{modulation}$), dans lequel la fréquence de modulation ($f_{modulation}$) est inférieure à la fréquence fondamentale et est d'au moins 100 kHz, et dans lequel le signal de sortie (a) peut être modulé par une modulation d'impulsion en largeur de manière à ce qu'un facteur de crête (CF) du signal de sortie modulé (a1,..., a4) approprié pour une application spécifique de l'appareil chirurgical à haute fréquence (1) soit obtenu,
**caractérisé en ce qu'**un facteur de crête (CF) peut être ajusté dans un intervalle de 5 à 11 ou supérieur à 15.

2. Appareil chirurgical à haute fréquence selon la revendication 1,
**caractérisé en ce que** la fréquence de modulation ($f_{modulation}$) et la fréquence fondamentale ($f_{générateur}$) sont constantes.

3. Appareil chirurgical à haute fréquence selon la revendication 1,
**caractérisé en ce que** la fréquence de modulation ($f_{modulation}$) est variable et **en ce que** la fréquence fondamentale ($f_{générateur}$) est constante pour la modification du facteur de crête.

4. Appareil chirurgical à haute fréquence selon l'une quelconque des revendications précédentes,
**caractérisé en ce que** la fréquence de modulation est supérieure à 200 kHz.

5. Appareil chirurgical à haute fréquence selon l'une quelconque des revendications précédentes,
**caractérisé en ce que** la fréquence fondamentale ($f_{générateur}$) est supérieure à cinq fois la fréquence de modulation ($f_{modulation}$).

Fig. 1

Fig. 2

Fig. 3

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008004241 A1
- EP 0194078 A2